(19) Europäisches Patentamt
European Patent Office
Office européen des brevets

(11) EP 0 657 167 B1

(12) **FASCICULE DE BREVET EUROPEEN**

(45) Date de publication et mention de la délivrance du brevet:
**22.03.2000 Bulletin 2000/12**

(51) Int. Cl.[7]: **A61K 31/70**

(21) Numéro de dépôt: **94402575.8**

(22) Date de dépôt: **15.11.1994**

(54) **Application de l'oenothéine B pour obtenir un médicament destiné au traitement des troubles liés à l'hyperandrogénie.**

Verwendung von Oenothein B zur Herstellung eines Medikaments zur Behandlung der durch Hyperandrogenität bedingte Störungen.

Use of oenotheine B for the manufacture of a medicament for the treatment of disorders related to hyperandrogenism.

(84) Etats contractants désignés:
**CH DE FR GB IT LI NL**

(30) Priorité: **16.11.1993 FR 9313623**

(43) Date de publication de la demande:
**14.06.1995 Bulletin 1995/24**

(60) Demande divisionnaire:
**99200220.4 / 0 947 522**

(73) Titulaire:
**HOECHST MARION ROUSSEL**
**92800 Puteaux (FR)**

(72) Inventeurs:
- **Gourvest, Jean-François**
  **F-77410 Claye-Souilly (FR)**
- **Kasal, Alexander**
  **101 00 Prague 10 (CZ)**
- **Lesuisse, Dominique**
  **F-75018 Paris (FR)**
- **Teutsch, Jean-Georges**
  **F-93500 Pantin (FR)**

(74) Mandataire:
**Vieillefosse, Jean-Claude et al**
**Hoechst Marion Roussel**
**Département des Brevets**
**102, Route de Noisy**
**93235 Romainville Cédex (FR)**

(56) Documents cités:
**EP-A- 0 297 547**

- **BIOLOGICAL AND PHARMACEUTICAL BULLETIN, vol. 16, no. 4, Avril 1993 pages 379-387, MIYAMOTO, K. ET AL 'ANTITUMOR ACTIVITIES OF ELLAGITANNINS AGAINST SARCOMA-180 IN MICE'**
- **JAPANESE JOURNAL OF CANCER RESEARCH, vol. 84, no. 1, Janvier 1993 pages 99-103, MIYAMOTO, K. ET AL 'ANTITUMOR ACTIVITY OF OENOTHEIN B, A UNIQUE MACROCYCLIC ELLAGITANNIN'**
- **DATABASE WPI Week 8902 Derwent Publications Ltd., London, GB; AN 89-008823 & EP-A-0 297 547 (LEAD CHEM CO LTD) , 4 Janvier 1989**
- **DATABASE WPI Week 8929 Derwent Publications Ltd., London, GB; AN 87-201982 & JP-A-62 129 220 (LEAD CHEMICAL CO.) , 11 Juin 1987**
- **JOURNAL OF LIQUID CHROMATOGRAPHY, vol. 13, no. 18, 1990 pages 3637-3650, OKUDA, T. ET AL 'FRACTIONATION OF PHARMACOLOGICALLY ACTIVE PLANT POLYPHENOLS BY CENTRIFUGAL PARTITION CHROMATOGRAPHY'**
- **SCIENTICA PHARMACEUTICA, vol. 51, 1983 pages 158-167, HIERMANN, A. 'DIE UNTERSUCHUNG POTENTIELLER WIRKSTOFFE IN EPILOBIUM-ARTEN'**
- **SCIENTICA PHARMACEUTICA, vol. 53, 1985 pages 39-44, HIERMANN, A. ET AL 'DIE UNTERSUCHUNG POTENTIELLER WIRKSTOFFE IN EPILOBIUM-ARTEN'**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Xerox (UK) Business Services
2.16.7/3.6

## Description

La présente invention concerne l'application de l'oenotheine B pour obtenir un médicament destiné au traitement des troubles liés à l'hyperandrogénie.

Il existe environ 200 espèces différentes d'Epilobium (famille des Onagreaceae). L'Epilobium Parviflorum ainsi que d'autres espèces d'Epilobium sont décrits en médecine traditionnelle comme bénéfiques à toutes sortes de désordres tels que ceux de la vessie, du rein et plus particulièrement de la prostate (A. Hiermann, H. Juan et W. Sametz, J. Ethnopharmacol. 17 (1986) 161-167 ; V.E. TYLER Pharm. Int. (1986) 205).

Les constituants d'Epilobium sont multiples et de structures complexes. Plusieurs analyses ont été tentées à ce jour et mettent en évidence divers types de composés. Toutefois, la composition demeure mal connue.

On a ainsi trouvé la présence de stérols (A. Hiermann , Sci. Pharm. 51 (1985) 39), de triterpènes et de flavonoides (flavonol aglycones et glycosides) (A. Hiermann et K. Mayr, Sci. Pharm. 51 (1998) 158). Récemment, la séparation des flavonols glycosides de l'Epilobium Parviflorum a permis de mettre en évidence la présence de la quercitrine, de la myricitrine, de l'isomyricitrine, d'acide gallique et d'acide chlorogénique, (I. Slacanin, A. Marston, K. Hostettmann, N. Delabays et C. Darbellay J. Chrom. 557 (1991) 391-398).

La demanderesse vient de découvrir que l'Epilobium Parviflorum renfermait en outre un autre composé particulièrement intéressant jamais isolé à ce jour de cette plante, l'oenotheine B.

L'oenotheine B est un tannin oligomérique hydrolysable. Il a été isolé pour la première fois des feuilles d'Oenothera erythrosepala Borbas (T. Hatano et al J. Chem. Soc., Perkin Trans. 1, (1990), 2735 ; 104th Annual Meeting of the Pharmaceutical Society of Japan, Mars 1984, Sendai et Chem. Pharm. Bull. (1989) 37(8) 2269-2271).

L'oenotheine B a été ensuite trouvée dans différentes plantes Onagracées et dans Woodfordia fruticosa (T. Yoshida et al. Chem. Pharm. Bull. 38, 1211 (1990) qui fait partie de la famille des Lythraceae. Enfin, récemment, l'oenotheine B a été isolée à partir du fruit séché de l'Eucalyptus Alba REINW qui fait partie de la famille des Myrtaceae (T. Yoshida et al. Chem. Pharm. Bull. (1992) 40(7) 1750-1754).

Bien que découverte à une époque récente, l'oenotheine B a fait l'objet d'études nombreuses qui ont démontré son intérêt en tant que médicament. L'oenotheine B possède ainsi un certain nombre de propriétés biologiques et pharmacologiques dans le domaine antiviral et antitumoral.

Activité antitumorale :

Lead Chemical Co J62129220 (11/06/87) Pr : 85JP-270494 (30/11/85)
Nippon Kayaku Co J03123793 (27/05/91) Pr : 89JP-258708 (05/10/89)
Ken-ichi Miyamoto et ai. Biol. Pharm. Bull. 16(4) 379-387 (1993)
A. Kuramochi-Motegi et al. Biochem. Pharm. 44(10) 1961-1965 (1992)
Yan-Jyu Tsai et al. The Journal of Biological Chemistry 267(20) 14436-14442
Ken-ichi Miyamoto et al. Jpn. J. Cancer Res. 84, 99-103 (1993).

Activité antivirale (Herpes):

K. Fukuchi etr al. Antiviral Research 11 (1989) 285-298

Activité antivirale (HIV) :

Lead Chemical Co EP-297547 (04/01/89) Pr 87JP-161572 (29/06/87).

C'est lors de l'étude d'un extrait particulier (fraction aqueuse) d'une décoction d'Epilobium Parviflorum, que la demanderesse a observé que le dit extrait répondait de manière significative comme inhibiteur de l'enzyme testostérone 5-$\alpha$-réductase, une enzyme clé dans la biosynthèse de certains androgènes, en particulier la 4,5-$\alpha$-dihydrotestostérone (DHT).

Cette observation a conduit la demanderesse à tenter d'identifier la molécule responsable de cette inhibition. Celle-ci s'est révélée être l'oenothéine B.

En résumé, c'est donc la première fois que l'on isole et identifie l'oenotheine B comme l'un des principes actifs de l'Epilobium Parviflorum.

Et c'est la première fois que l'on détermine que l'oenotheine B agit en tant qu'inhibiteur de l'enzyme testostérone 5-α-réductase ce qu'en aucune manière l'art antérieur ne laissait prévoir.

L'oenotheine B présente une activité spécifique en tant qu'inhibiteur de la 5-α-réductase. La 5-α-réductase est une enzyme responsable de la conversion de la Testostérone en dihydrotestostérone (DHT), plus affine que la Testostérone sur le récepteur des androgènes (RA).

Cette hormone est essentielle chez le mâle, au développement in utéro des organes sexuels externes et ensuite dès la puberté, à l'apparition de certaines caractéristiques sexuelles secondaires (système pileux, visage et corps).

On retrouve la 5-α-réductase essentiellement dans la prostate et dans la peau, là où il semble que la Testostérone doive être transformée en DHT pour agir.

Un excès de DHT peut être responsable d'acné ou d'alopécie androgénique, d'hirsutisme chez la femme, et son accumulation au niveau de la prostate, d'hyperplasie bénigne (HBP) ou de cancer de la prostate. On estime que 80 % des hommes de plus de 80 ans présentent une HBP et 70 % des foyers cancéreux.

L'inhibition de cette étape enzymatique dans le métabolisme stéroïdien entraîne une chute du niveau de DHT in vivo. Cette réduction du niveau de DHT a pour effet de réduire la taille de la prostate chez l'homme et donc a un effet bénéfique dans le traitement de l'hyperplasie bénigne de la prostate, du cancer de la prostate, et de certaines maladies androgéno-dépendantes.

Un traitement inhibant la 5-α-réductase est donc très utile et ne présente pas les inconvénients d'un antiandrogène qui bloque le récepteur.

Cette propriété inhibitrice de la 5-α-réductase rend donc l'oenotheine B apte à être utilisée dans le traitement des troubles liés à l'hyperandrogénie, notamment celui de l'hyperplasie bénigne de la prostate (HBP), du cancer de la prostate, de l'acné, de l'alopécie androgénique, de la séborrhée, ou encore de l'hirsutisme féminin.

La présente invention a donc pour objet, l'application de l'oenotheine B pour obtenir un médicament destiné au traitement des troubles liés à l'hyperandrogénie.

La posologie varie en fonction de l'affection à traiter et de la voie d'administration.

Elle peut varier par exemple de 5 à 100 mg/kg et, de préférence, de 10 à 20 mg/kg et par jour chez l'adulte par voie orale.

L'oenotheine B peut donc être employée pour préparer des compositions pharmaceutiques la contenant à titre de principe actif.

L'oenotheine B selon l'invention peut être utilisée par voie digestive, parentérale ou locale, par exemple par voie transdermique. Elle peut être prescrite sous forme de comprimés simples ou dragéifiés, de gélules, de granulés, de suppositoires, de préparations injectables, d'ovules, de pommades, de crèmes, de gels, de patchs, lesquels sont préparés selon les méthodes usuelles.

Le principe actif peut y être incorporé à des excipients habituellement employés dans ces compositions pharmaceutiques tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou emulsifiants, les conservateurs.

**Etude pharmacologique de l'oenotheine B**

Le test de mesure de l'activité 5-α-réductase se fait in-vitro en incubant l'enzyme et son substrat (la Testostérone) et en mesurant par chromatographie HPLC la quantité de métabolites 5-α-réduits formés (dihydrotestostérone (DHT) et 5-α-androstane-diol).

On utilise des homogénats de prostates humaines obtenues par prostatectomies radicales pour cause d'hyperplasie bénigne de la prostate (HBP).

Méthode

Préparation de l'homogénat :

La prostate est recueillie dès la sortie du bloc opératoire et emballée dans du papier aluminium placé dans un récipient rempli de glace carbonique. Au laboratoire, si l'homogénat n'est pas préparé immédiatement, la prostate peut être conservée à -80°C.

On émince la prostate sur de la glace en très petits morceaux, puis on broie dans un milieu A (20 mM de phosphate de potassium, pH 6,5 ; 0,32 M de sucrose ; 1 mM de dithiotréitol (DTT) ; 50 μM de sel de sodium hydraté de triphosphopyridine nucléotide (NADPH), au polytron puis au potter verre-verre. Après centrifugation à 140000 g pendant 60 mn à 4°C, on remet en suspension dans 10 à 20 ml de milieu B (20 nM de phosphate de potassium, pH 6,5 ; 20 % de glycérol ; 1 mM de dithiotréïtol). On répartit dans des tubes Nunc et on conserve à -80°C.

On effectue un dosage des protéines sur un peu d'homogénat pour son utilisation future dans le test et on s'assure ainsi d'avoir au moins 10 mg de protéines/ml d'homogénat.

Mesure de l'activité 5-α-réductase :

L'incubation se tait à pH 5,5 à raison de 1 ml de milieu/tube, en présence de citrate (milieu normal de la prostate).

1) Préparation du substrat (S) c'est-à-dire du mélange de Testostérone "froide" et de Testostérone "tritiée" avec une dilution isotopique de 100 dans un tampon citrate trisodique pH 5,0 :

a) tampon citrate tri-sodique 40 mM : On pèse 11,76 g de citrate-3Na, $2H_20$ (Merk ref. Art 6448) que l'on dissout dans 1 litre d'eau distillée (Milli-Q) et on ajuste à pH 5,0.

b) mélange de Testostérone "froide" et de Testostérone "tritiée" avec une dilution isotopique de 100 : Pour obtenir une solution de testostérone de 1 mM, on pèse 2,88 mg de Testostérone "froide" que l'on dissout dans 10 ml d'éthanol. Cette solution est diluée au 0,99/1000 soit 9,9 µl dans 10 ml de tampon citrate (= solution $10^{-6}$M). On ajoute 9 µl de Testostérone-$^3$H (NET-370) à 10 ml de la solution précédente (= dilution isotopique de 100). Le substrat (S) est prêt.

2) Préparation du mélange Enzyme +DTT +NADPH

a) Tampon phosphate de potassium, 40 mM, pH 6,5 : On pèse 5,44 g de $KH_2PO_4$ (Riedel de Haen ref. 30407) que l'on dissout dans 1 l d'eau distillée (Milli-Q) et on ajuste à pH 6,5.

b) DTT 1 mM (Sigma)
On pèse 3,1 mg de DTT et on dissout dans 1 ml de tampon phosphate.

c) NADPH 500 µM (Sigma)
On pèse 8,33 mg de NADPH et on dissout dans 1 ml de tampon phosphate.

d) Mélange enzyme+DTT+NADPH (E) :
Pour un essai, on mélange à 4°C, 0,5 mg de protéines de l'homogénat de prostate, 50 µl de DTT et 50 µl de NADPH. On complète à 500 µl avec le tampon phosphate.

3) Incubation :
On prépare autant de tubes que d'essais. Dans chaque tube, on met 500 µl de (S) et 10 µl d'inhibiteur à la concentration voulue (ou d'éthanol pour les témoins).
On chauffe au bain-marie les tubes contenant le mélange (S) et la fiole contenant le mélange (E) à préincuber pendant 3 à 5 mn.
Pour démarrer la réaction, on transfère rapidement 500 µl de E dans chaque tube contenant S + l'inhibiteur et on incube à 37°C avec une légère agitation pendant 30 mn. La réaction est stoppée à la fin de l'incubation en mettant les tubes dans de la glace.

4) Extraction
Aussitôt la réaction stoppée, on met 2 ml d'acétate d'éthyle dans chaque tube et on agite au Multi-tubes vortexer pendant 1 mn. On laisse décanter pendant 10 mn puis on récupère dans d'autres tubes 1,6 ml de la phase organique (supérieure) de l'extrait (= 80 %). Après évaporation totale de l'acétate d'éthyle, on ajoute dans chaque tube 800 µl de la phase mobile qui servira dans l'HPLC.

5) Chromatographie HPLC :
La mesure des taux de métabolites produits au cours de l'incubation est effectuée en séparant les composés formés par chromatographie liquide à haute performance (HPLC) en phase inverse. On utilise une colonne ODS-Hypersil (diamètre des particules = 5 µm) et une phase mobile composée de MeOH/THF/$H_20$ dans un rapport de 45/15/40. L'extrait recueilli après extraction est injecté automatiquement au niveau de la chaine HPLC et grâce au système de mesure de radioactivité en ligne (Berthold), on n'apprécie que les métabolites radioactifs, donc formés à partir de la Testostérone marquée du substrat. Cette mesure est très sensible et élimine tous les produits endogènes éventuels.

Expression des résultats et méthodes de calcul :

Les taux de métabolites formés sont calculés par rapport à une courbe étalon rentrée dans le logiciel MT2 de l'HPLC. Au niveau du MT2, ils sortent en pmoles/µl injectés. Des courbes étalons existent pour la testostérone, la DHT et l'androstane-diol, les deux métabolites 5-α-réduits de la testostérone.

Les taux sont exprimés en moles de produits formés/mg de protéines d'homogénat, chaque essai étant effectué en double, et on calcule le pourcentage d'activité 5-α-réductase résiduelle par rapport aux essais témoins :

$$\% = \frac{\text{Taux de produits formés en présence d'inhibiteur x 100}}{\text{Taux de produits formés pour les témoins}}$$

<u>Résultat</u>

La $CI_{50}$ d'inhibition de l'activité 5-α-réductase du témoin pour l'oenotheine B est de $2{,}2.10^{-5}$M.

**Revendications**

1. L'application de l'oenotheine B pour obtenir un médicament destiné au traitement des troubles liés à l'hyperandrogénie.

**Claims**

1. The use of oenotheine B to obtain a medicament intended for the treatment of disorders linked to hyperandrogenism.

**Patentansprüche**

1. Verwendung von Oenothein B zur Herstellung eines Medikaments zur Behandlung von Störungen im Zusammenhang mit Hyperandrogenie.